# EUROPEAN PATENT SPECIFICATION

(11) **EP 2 888 237 B1**
(45) Date of publication and mention of the grant of the patent: **18.10.2017**
(21) Application number: 13831274.9
(22) Date of filing: 20.08.2013
(51) Int. Cl.: C07D 251/14, C07D 277/20, C07D 417/06

(54) **METHOD FOR PREPARATION OF THIAZOLE DERIVATIVES**
VERFAHREN ZUR HERSTELLUNG VON THIAZOLDERIVATEN
PROCÉDÉ POUR LA PRÉPARATION DE DÉRIVÉS DE THIAZOLE

(30) Priority: 21.08.2012 CN 201210299527
(43) Date of publication of application: 01.07.2015
(73) Proprietor: DSM IP Assets B.V., 6411 TE Heerlen (NL)
(72) Inventor: PENG, Kun, Shanghai, 201203 (CN); DAI, Xixiang, Shanghai, 201203 (CN); ZHAO, Lizhi, Shanghai, 201203 (CN); CAO, Weifeng, Shanghai, 201203 (CN); LETINOIS, Ulla, Basel, 4002 Kaiseraugst (CH)
(74) Representative: Kurt, Manfred
(86) International application number: PCT/CN2013/081848
(87) International publication number: WO 2014/029320

(56) References cited:
- WO-A1-2005/095391
- GB-A- 501 476
- JP-A- S4 975 588
- JP-A- S63 227 578
- DATABASE CAPLUS [Online] CHEMICAL ABSTRACTS SERVICE, COLUMBUS, OHIO, US; 12 May 1984 (1984-05-12), XP002751402, retrieved from STN Database accession no. 1975:156270 -& DATABASE REGISTRY CHEMICAL ABSTRACTS SERVICE, COLUMBUS, OHIO, US; 16 November 1984 (1984-11-16), XP002751403, Database accession no. 55289-66-2 & JP 49 075588 S (TAKEDA CHEM IND. LTD.) 20 July 1974 (1974-07-20)
- Pierre Contant ET AL: "127. A New Convergent Synthesis of Thiamine Hydrochloride", , vol. 73 1990, pages 1300-1305, XP055182389, Retrieved from the Internet: URL:http://onlinelibrary.wiley.com/store/1 0.1002/hlca.19900730518/asset/19900730518_ ftp.pdf?v=1&t=i8fnagy0&s=9e5ab2c2cc8ca336b 7b197c760c2b2c81cb1cf8e [retrieved on 2013]
- CONTANT, PIERRE ET AL.: 'A New Convergent Synthesis of Thiamine Hydrochloride.' HELVETICA CHIMICA ACTA. vol. 73, 1990, pages 1300 - 1305, XP055182389

## Description

### Technical filed

The present invention is related to a method for synthesizing thiazole derivatives, in particular, is related to a method for the preparation of thiazole rings.

### Background of the Invention

Thiazole derivatives have various medical uses and biological activities. For example, clomethiazole can be used as sedative and hypnotic drugs and tiazofurine can be used as anticancer drugs. In addition, thiazole rings are an important component of vitamin B₁, and thiazole derivatives, such as 4-methyl-5-(2-hydroxyethyl)thiazole, are important intermediates for preparing vitamin B₁.

Currently, thiazole derivatives are synthesized mainly by Hantasch process in which thiazole ring is synthesized by reacting haloketone with thiourea. However, the process has some disadvantages including toxic solvents used, expensive catalysts needing, high energy consumption, low yield, severe reaction conditions, and difficult post-processing, *etc..* (E. Aguilar, et al., Tetrahetro Lett., 35: 2473-2476 (1994); Yin-zhu WANG, et al., Material Report A: Review part, Jan., 2012, 26(1):71-78) Contant, Pierre et. al.: "A new convergent synthesis of thiamine hydrochloride", Helvetica Chimica Acta, vol. 73, 1990, pages 1300-1305 discloses the preparation of thiamine hydrochloride by reacting an α-mercaptoketone with an amine. Therefore, a novel method for synthesizing thiazole derivatives is needed.

### Summary of the Invention

The present invention provides a novel method for preparing thiazole derivatives. The method has a high yield, easily-controlled reaction conditions and does not use any catalyst toxic or hazardous to environment, so it is economic, environmental friendly and suitable for industrial production.

In particular, the present invention provides a method for the preparation of a thiazole compound of formula (I), 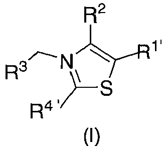 comprising reacting a compound of formula (II) with a compound of formula (III) in a solvent containing formic acid, 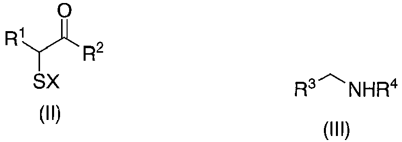 wherein,
each of R¹, R^{1'} and R² is independently hydrogen, or substituted or unsubstituted C₁-C₁₀ hydrocarbyl;
R³ is H, or substituted or unsubstituted C₁-C₁₀ hydrocarbyl, 5- or 6-members heterocyclic group, or 5- or 6-members heteroaryl;
R⁴ is hydrogen, or substituted or unsubstituted C₁-C₁₀ acyl;
R^{4'} is hydrogen, or substituted or unsubstituted C₁-C₁₀ hydrocarbyl; and
X is H, C₁-C₁₀ acyl, or the following structure:

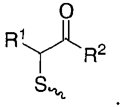

### Detailed Description of the Invention

In the present invention, the "C₁-C₁₀ hydrocarbyl" refers to linear chain or branched chain, saturated or unsaturated, cyclic or non-cyclic hydrocarbyl comprising 1-10 carbon atoms, including but not limited to alkyl, alkenyl, alkynyl, and aryl such as phenyl. Preferably, the "C₁-C₁₀ hydrocarbyl" is C₁-C₄ hydrocarbyl, for example but not limited to methyl, ethyl, propyl, isopropyl, cyclopropyl, butyl, isobutyl, tert-butyl, methyl cyclopropyl, cyclobutyl, ethenyl, propenyl, butenyl, ethynyl, propynyl and butynyl. More preferably, the "C₁-C₁₀ hydrocarbyl" is methyl, ethyl or phenyl.

In the present invention, the "C₁-C₁₀ alkyl" refers to linear chain or branched chain, cyclic or non-cyclic saturated alkyl comprising 1-10 carbon atoms. Preferably, the "C₁-C₁₀ alkyl" is C₁-C₄ alkyl, for example but not limited to methyl, ethyl, propyl, isopropyl, cyclopropyl, butyl, isobutyl, tert-butyl, methyl cyclopropyl and cyclobutyl. More preferably, the "C₁-C₁₀ alkyl" is methyl or ethyl.

In the present invention, the "5- or 6-members heterocyclic group" refers to saturated or unsaturated, non-aromatic, monocyclic or multicyclic ring system comprising five or six ring atoms, which comprise carbon atom, and one or more heteroatoms selected from the group consisting of nitrogen, oxygen and/or sulphur. The examples of the "5- or 6-members heterocyclic group" include but are not limited to pyrrolidinyl, piperazinyl, piperidinyl, morpholinyl, tetrahydrofuranyl, tetrahydropyranyl, *etc..*

In the present invention, the "5- or 6-members heteroaryl" refers to aromatic ring comprising five or six ring atoms, which comprise carbon atom, and one or more heteroatoms selected from the group consisting of nitrogen, oxygen, and/or sulphur. The examples of the "5- or 6-members heteroaryl" include but are not limited to furyl (e.g., 2-furyl, 3-furyl, *etc.),* thienyl (e.g., 2-thienyl, 3-thienyl, *etc.),* pyrrolyl (e.g., 1-pyrrolyl, 2-pyrrolyl, 3-pyrrolyl *etc.),* pyridyl (e.g., 2-pyridyl, 3-pyridyl, 4-pyridyl, *etc.),* pyrazinyl, pyridazinyl (e.g., 3-pyridazinyl, 4-pyridazinyl, *etc.),* pyrimidinyl (e.g., 2-pyrimidinyl, 4-pyrimidinyl, 5-pyrimidinyl, *etc.),* triazolyl (e.g., 1,2,3-triazolyl, 1,2,4-triazolyl, *etc.),* tetrazolyl (e.g. 1H-tetrazolyl, 2H-tetrazolyl, *etc.),* thiazolyl (e.g., 2-thiazolyl, a 4-thiazolyl, 5-thiazolyl, *etc.),* pyrazolyl (e.g., 3-pyrazolyl, 4-pyrazol, *etc.),* oxazolyl (e.g., 2-oxazolyl, 4-oxazolyl, 5-oxazolyl, *etc.),* isoxazolyl (e.g., 3-isoxazolyl, 4-isoxazolyl, 5-isoxazolyl, *etc.),* isothiazolyl (e.g., 3-isothiazolyl, 4-isothiazolyl, 5-isothiazolyl, *etc.),* oxadiazolyl and thiadiazolyl, *etc..*

In the present invention, the "C₁-C₁₀ acyl" refers to the group with the formula of RC(O)-, wherein R is H; or linear chain or branched chain, saturated or unsaturated, cyclic or non-cyclic hydrocarbyl comprising 1-9 carbon atoms, including but not limited to alkyl, alkenyl, alkynyl and aryl. Preferably, the "C₁-C₁₀ acyl" is C₁-C₄ acyl, for example but not limited to formyl, acetyl, propionyl, butyl and isobutyryl. More preferably, the "C₁-C₁₀ acyl" is formyl or acetyl.

In the present invention, the "substituted" refers to that the modified group thereof may be substituted by one or more of the following substituents: hydroxyl, amino, halogen (e.g., fluorine, chlorinum, bromium or iodine), C₁-C₁₀ hydrocarbyl, C₁-C₁₀ acyl, C₁-C₁₀ hydrocarbyloxyl and C₁-C₁₀ acyloxyl, wherein the "C₁-C₁₀ hydrocarbyloxyl' refers to the group with the structure of C₁-C₁₀ hydrocarbyl-O and the "C₁-C₁₀ hydrocarbyl" is defined as above, the "C₁-C₁₀ acyloxy" refers to the group with the structure of C₁-C₁₀ acyl-O and the "C₁-C₁₀ acyl" is defined as above. Any person skilled in the art should understand that the substituents of the present invention can optionally be further substituted by one or more other aforementioned substituents. For example, hydroxyl as a substituent may be further substituted by C₁-C₁₀ hydrocarbyl or C₁-C₁₀ acyl.

The invention provides a novel method for the preparation of a thiazole compound of formula (I), 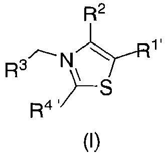 comprising reacting a compound of formula (II) with a compound of formula (III) in a solvent containing formic acid,

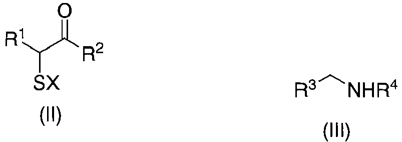

Wherein,
Each of R¹ and R^{1'} is independently hydrogen; or substituted or unsubstituted C₁-C₁₀ hydrocarbyl, such as C₁-C₁₀ hydrocarbyl substituted by hydroxyl which may optionally be substituted by C₁-C₁₀ acyl. Preferably, each of R¹ and R¹ is independently HO-C₁-C₁₀ hydrocarbyl or C₁-C₁₀ acyl-O-C₁-C₁₀ hydrocarbyl.

R¹ and R^{1'} can be the same or different. For example, R¹ and R^{1'} are the same when R¹ is hydrogen, or unsubstituted C₁-C₁₀ hydrocarbyl; and R¹ and R^{1'} are the same or different when R¹ is C₁-C₁₀ acyl-O-C₁-C₁₀ hydrocarbyl, for example, when R¹ is acetyl-O-C₁-C₁₀ hydrocarbyl, R^{1'} may be formyl-O-C₁-C₁₀ hydrocarbyl, acetyl-O-C₁-C₁₀ hydrocarbyl or HO-C₁-C₁₀ hydrocarbyl.

R² is hydrogen, or substituted or unsubstituted C₁-C₁₀ hydrocarbyl, preferably, C₁-₁₀ alkyl.

R³ is H, or substituted or unsubstituted C₁-C₁₀ hydrocarbyl, 5- or 6-members heterocyclic group or 5- or 6-members heteroaryl. Preferably, R³ is pyrimidinyl optionally substituted by C₁-C₁₀ hydrocarbyl such as methyl and/or amino, for example, R³ is 2-methyl-4-amino-5-pyrimidinyl.

R⁴ is hydrogen, or substituted or unsubstituted C₁-C₁₀ acyl;

R^{4'} is hydrogen, or substituted or unsubstituted C₁-C₁₀ hydrocarbyl.

X is H, C₁-C₁₀ acyl, or the following structure:

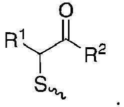

In an embodiment, the present invention is related to a method for the preparation of a thiazole compound of formula (I), wherein,
each of R¹ and R^{1'} is independently methyl, ethyl, propyl, ethenyl, hydroxyethyl, formyloxyethyl or acetyloxyethyl;
R² is methyl, ethyl, ethenyl, hydroxymethyl or hydroxyethyl;
R³ is phenyl, benzyl, 2-methyl-pyrimidinyl, 4-amino-pyrimidinyl, or 2-methyl-4-amino-pyrimidinyl;
R⁴ is H, formyl or acetyl;
R^{4'} is H or methyl; and
X is H, formyl, acetyl, or the following structure:

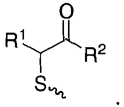

In a preferred embodiment, the present invention provides a method for the preparation of a thiazole compound of formula (Ia), 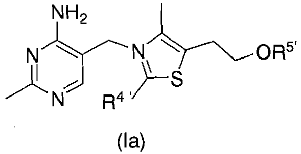 comprising reacting a compound of formula (IIa) with a compound of formula (IIIa) in a solvent containing formic acid, 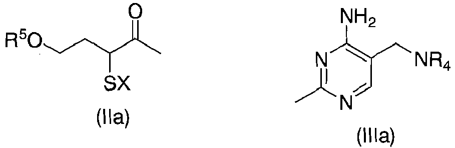 wherein R⁴ and R^{4'} are defined as above, and each of R⁵ and R^{5'} is independently hydrogen, formyl or acetyl, and preferably, R⁵ is formyl or acetyl, and R^{5'} is formyl, acetyl or hydrogen.

In another preferred embodiment, the present invention provides a method for preparing a thiazole compound of formula (Ia), 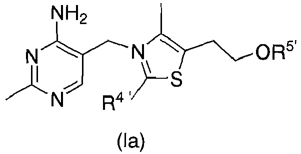 comprising reacting a compound of formula (IIb) with a compound of formula (IIIa) in a solvent containing formic acid, 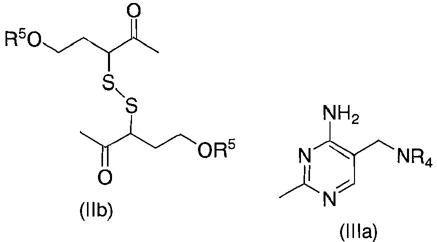 wherein R⁴ and R^{4'} are defined as above, and each of R⁵ and R^{5'} is independently hydrogen, formyl or acetyl, and preferably, R⁵ is formyl or acetyl, and R⁵ is formyl, acetyl or hydrogen.

According to the method of the present invention, the solvent containing formic acid may be formic acid or solvents mixture containing formic acid. The solvents mixture may further contain other solvent known in the art, such as acetic acid, dioxane and/or dimethylformamide, *etc..*

According to the method of the present invention, the reaction may be carried out at 80-150°C, preferably at 100-120°C, more preferably at reflux temperature. The reaction may be carried out under an atmosphere pressure or an increased pressure or a reduced pressure. It should be easy for one person skilled in the art to determine the pressure suitable for the reaction of the present invention according to the conventional methods known in the art.

According to the method of the present invention, the compound of formula (II)/formula (IIa)/formula (IIb) and the compound of formula (III)/formula (IIIa) as raw materials may react in any ratio. However, preferably, the molar ratio between the compound of formula (II)/formula (IIa)/formula (IIb) and the compound of formula (III)/formula (IIIa) added into the reaction is from 1:2 to 2:1, more preferably from 1:1 to 2:1, further more preferably from 1:1 to 1.4:1, and most preferably is 1.1:1.

According to the method of the present invention, the reaction is carried out in batches or continuously. In the embodiment where the reaction is carried out in batches, the compound of formula (II)/ formula (IIa)/formula (IIb) and the compound of formula (III) /formula (IIIa) may be added simultaneously or successively in any order, for example, added dropwise into the reaction, in one time or in portions or continuously. Preferably, the compound of formula (II)/formula (IIa)/formula (IIb) and/or the compound of formula (III)/formula (IIIa) are added into the reaction in batches, such as in two, three, four, five, six, seven, eight or more batches; or continuously. In the embodiment where the reaction is carried out continuously, the compound of formula (II)/formula (IIa)/formula (IIb) and the compound of formula (III)/formula (IIIa) may be added into the reaction continuously.

The reaction of the present invention produces water as a byproduct, so any person skilled in the art should understand that adding additional water absorbent will be beneficial for the reaction. The water absorbent suitable for the present invention includes, but is not limited to, orthoformate such as trimethyl orthoformate and triethyl orthoformate; acyl chloride such as acetyl chloride; anhydrides such as phosphoric anhydride, acetic anhydride, boric anhydride (B₂O₃) and so on; esters such as methyl formate. In addition, adding a catalyst would also be beneficial for the reaction. The examples of the catalyst suitable for the present invention include, but are not limited to, mineral acid such as sulfuric acid, hydrochloric acid, nitric acid, *etc.;* and phosphorus-containing catalysts such as phosphates, polyphosphoric acids, and polyphosphates. Any person skilled in the art should understand that the water absorbent and the catalyst are not necessary for the method of the present invention, and the method of the present invention can obtain the yield of over 87% even without using the water absorbent and/or the catalyst. Therefore, according to the method of the present invention, the reaction preferably is carried out in the absence of water absorbent and/or a catalyst.

In the method of the present invention, a reductant may be optionally added into the reaction. In particular, a reductant is preferably added into the reaction especially when X in formula (II)/formula (IIa) is 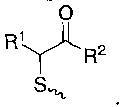 The examples of suitable reductants include but are not limited to metal reductant such as iron powder and zinc powder.

The compound of formula (II)/formula (IIa)/formula (IIb) are commercially available or can be synthesized by methods known in the art, for example, prepared by vulcanization of a corresponding chloride thereof. The compound of formula (III) or formula (IIIa) are commercially available or can be synthesized by methods known in the art, such as the methods described in JP 58-065 279, EP 0172515 A, EP 0001760 A, US 4226799, DE 3511273A and WO 2006/079504.

The method of the present invention is preferably carried out in an inert gas such as nitrogen or carbon monoxide environment. The obtained compound of formula (I)/formula (Ia) can be purified by using known methods such as crystallization and filtration, or used directly without purification into the next reaction, for example, used directly into the preparation of vitamin B₁.

The method of the present invention may be carried out without using environmentally polluting catalysts such as phosphorus-containing catalysts, even without using any catalyst, while obtaining a high yield of up to 87%. Accordingly, the method is more environment friendly and more economic over prior art.

The present invention is to be further described by the following examples. These examples are merely for illustrative purposes and should not in any way be interpreted as limiting the scope of the invention.

### Examples

### Example 1

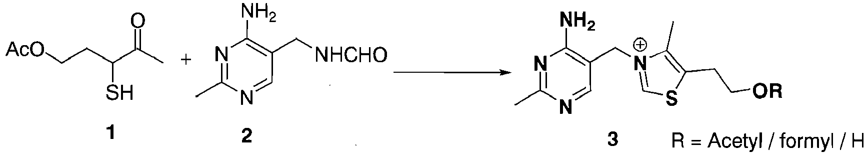

Under the protection of nitrogen, **150** mmol of compound **2** and **150** ml of formic acid were added into a three-necked flask equipped with a stirring device and heated to reflux. **180** mmol of compound **1** was added in five batches, and each batch was reacted for **1** hour before a next batch was added. After all the five batches were added, the reaction mixture was refluxed overnight, cooled down to room temperature, and then detected by quantitative liquid chromatography to obtain the following results.

| Conversion rate | Selectivity | Total yield | Yield of compound **3** (R=acetyl) | Yield of compound **3** (R=formyl) | Yield of compound **3** (R=H) |
|---|---|---|---|---|---|
| **93.8%** | **90.4%** | **84.8%** | **15.6%** | **60.4%** | **8.8%** |

### Example 2

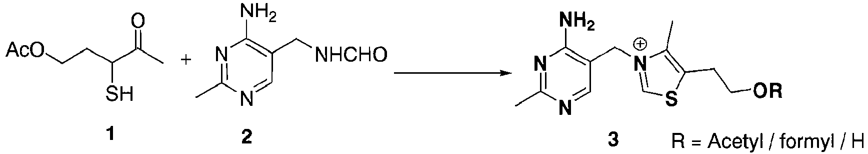

Under the protection of nitrogen, **30** mmol of compound **2, 45** ml of boric anhydride and **30** ml of formic acid were added into a three-necked flask equipped with a stirring device and heated to reflux. **37.5** mmol of compound **1** was added in five batches, and each batch was reacted for **1** hour before a next batch was added. After all the five batches were added, the reaction mixture was heated at reflux for 4 hours, cooled down to room temperature, and then detected by quantitative liquid chromatography to obtain the following results.

| Conversion rate | Selectivity | Total yield | Yield of compound **3** (R=acetyl) | Yield of compound **3** (R=formyl) | Yield of compound **3** (R=H) |
|---|---|---|---|---|---|
| **92.4%** | **98.1%** | **90.6%** | **45.4%** | **41.5%** | **3.7%** |

### Example 3

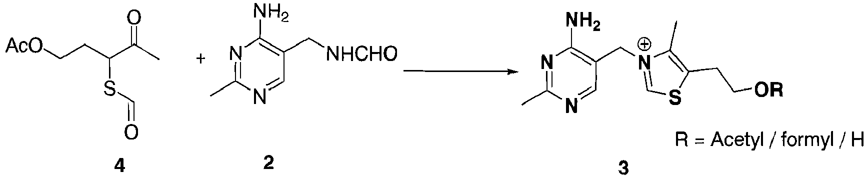

**150** mmol of compound **2, 380** ml of formic acid and **150** mmol of sulfuric acid solution (**98%**) were added into a three-necked flask equipped with a stirring device and heated to reflux. After the substrates were totally dissolved, **180** mmol of compound **4** was added in five batches, and each batch was reacted for **1** hour before a next batch was added. After all the five batches were added, the reaction mixture was refluxed overnight, cooled down to room temperature, and then detected by quantitative liquid chromatography to obtain the following results.

| Conversion rate | Selectivity | Total yield | Yield of compound **3** (R=acetyl) | Yield of compound **3** (R=formyl) | Yield of compound **3** (R=H) |
|---|---|---|---|---|---|
| **98.9%** | **94.6%** | **93.6%** | **23.3%** | **67.2%** | **3.1%** |

### Example 4

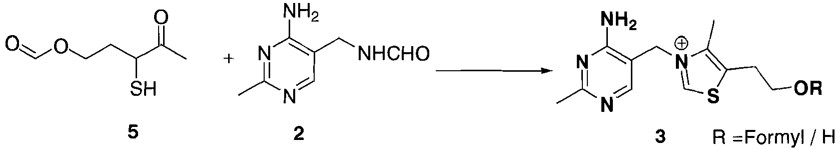

Under the protection of nitrogen, **11.36** mmol of compound **2** and **60** ml of formic acid were added into a three-necked flask equipped with a stirring device and heated to reflux. **13.63** mmol of compound **5** was added in six batches, and each batch was reacted for **1.5** hours before a next batch was added. After all the six batches were added, the reaction mixture was refluxed for **14** hours, cooled down to room temperature, and then detected by quantitative liquid chromatography to obtain the following results.

| Conversion rate | Selectivity | Total yield | Yield of compound **3** (R=formyl) | Yield of compound **3** (R=H) |
|---|---|---|---|---|
| **89.9%** | **97.2%** | **87.4%** | **86.1%** | **1.3%** |

### Example 5

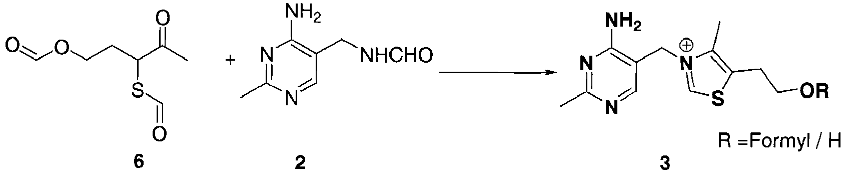

Under the protection of nitrogen, **11.36** mmol of compound **2, 60** ml of formic acid and **11.36** mmol of sulfuric acid solution (**98%**) were added into a three-necked flask equipped with a stirring device and heated to reflux. **13.63** mmol of compound **6** was added in six batches, and each batch was reacted for **1.5** hours before a next batch is added. After all the six batches were added, the reaction mixture was refluxed overnight, cooled down to room temperature, and then detected by quantitative liquid chromatography to obtain the following results.

| Conversion rate | Selectivity | Total yield | Yield of compound **3** (R=formyl) | Yield of compound **3** (R=H) |
|---|---|---|---|---|
| **90.5%** | **96.2%** | **87.1%** | **84.3%** | **2.8%** |

### Example 6

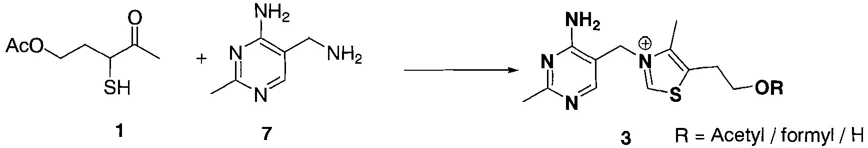

Under the protection of nitrogen, **150** mmol of compound **7** and **150** ml of formic acid were added into a three-necked flask equipped with a stirring device and heated to reflux. **180** mmol of compound **1** was added in five batches, and each batch was reacted for **1** hour before a next batch was added. After all the five batches were added, the reaction mixture was refluxed overnight, cooled down to room temperature, and then detected by quantitative liquid chromatography to obtain the following results.

| Conversion rate | Selectivity | Total yield | Yield of compound **3** (R=acetyl) | Yield of compound **3** (R=formyl) | Yield of compound **3** (R=H) |
|---|---|---|---|---|---|
| **92.2%** | **84.6%** | **78.0%** | **9.7%** | **60.3%** | **8.0%** |

### Example 7

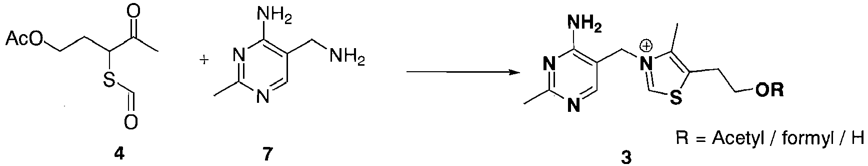

Under the protection of nitrogen, **150** mmol of compound **7** and **150** ml of **1** mol/L hydrogen chloride solution in formic acid were added into a three-necked flask equipped with a stirring device and heated to reflux. **180** mmol of compound **4** was added in five batches, and each batch was reacted for 1 hour before a next batch was added. After all the five batches were added, the reaction mixture was heated at reflux overnight, cooled down to room temperature, and then detected by quantitative liquid chromatography to obtain the following results.

| Conversion rate | Selectivity | Total yield | Yield of compound 3 (R=acetyl) | Yield of compound 3 (R=formyl) | Yield of compound 3 (R=H) |
|---|---|---|---|---|---|
| **95.0%** | **96.6%** | **91.8%** | **18.0%** | **68.5%** | **5.3%** |

### Example 8

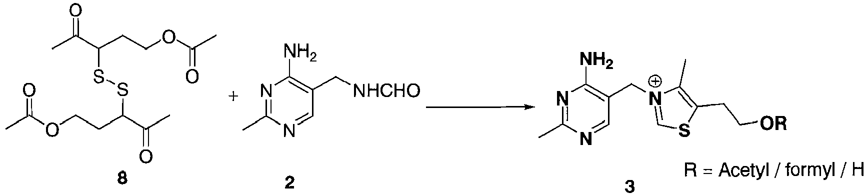

**0.62** mmol of compound **8, 1.20** mmol of compound **2** and **1.30** mmol of iron powder were added into a three-necked flask equipped with a stirring device and then **5** ml of formic acid was added. The mixture was heated to reflux with stirring. The reaction was monitored by LCMS. After the reaction was carried out for **40** hours, the following results were obtained by quantitative liquid chromatography.

| Conversion rate | Selectivity | Total yield | Yield of compound **3** (R=acetyl) | Yield of compound **3** (R=formyl) | Yield of compound **3** (R=H) |
|---|---|---|---|---|---|
| **46.8%** | **98.9%** | **46.3%** | **15.0%** | **29.7%** | **1.6%** |

### Example 9

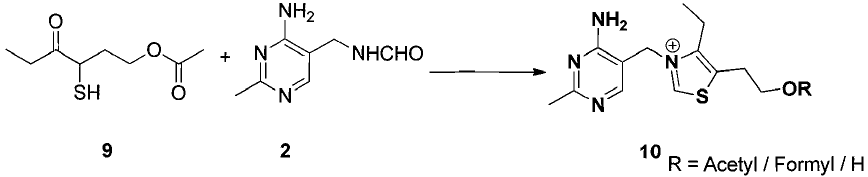

Under the protection of nitrogen, **5** mmol of compound **2** and **10** ml of formic acid were added into a three-necked flask equipped with a stirring device and heated to reflux. **4.5** mmol of compound **9** was added in three batches, and each batch was reacted for **1** hour before a next batch was added. After all the three batches were added, the reaction mixture was refluxed overnight, cooled down to room temperature, and then detected by LCMS to obtain the following results.

| Conversio n rate | Selectivity | Total yield | Yield of compound **10** (R=acetyl) | Yield of compound **10** (R=formyl) | Yield of compound **10** (R=H) |
|---|---|---|---|---|---|
| **47.8%** | **97.5%** | **46.6%** | **40.2%** | **5.1%** | **1.3%** |

### Example 10

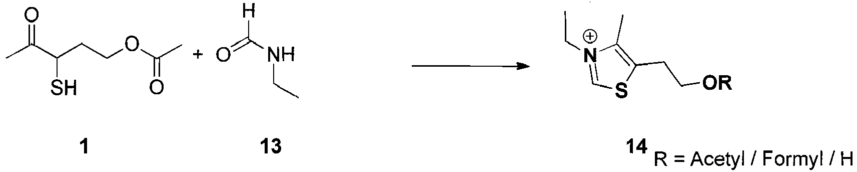

Under the protection of nitrogen, **39** mmol of compound **13** and **100** ml of formic acid were added into a three-necked flask equipped with a stirring device and heated to reflux. **45** mmol of compound **1** were added in six batches, and each batch was reacted for **1** hour before a next batch was added. After all the three batches were added, the reaction mixture was refluxed overnight, cooled down to room temperature, and then detected by LCMS to obtain the following results.

| Conversio n rate | Selectivity | Total yield | Yield of compound **14** (R=acetyl) | Yield of compound **14** (R=formyl) | Yield of compound **14** (R=H) |
|---|---|---|---|---|---|
| **92.3%** | **32.1%** | **29.6%** | **20.5%** | **9.1%** | **0%** |

### Example 11

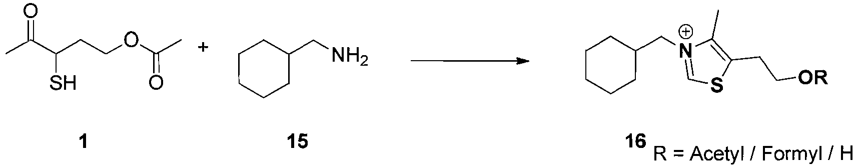

Under the protection of nitrogen, **18.4** mmol of compound **15** and **50** ml of formic acid were added into a three-necked flask equipped with a stirring device and heated to reflux. **22.7** mmol of compound **1** was added in six batches, and each batch was reacted for **1** hour before a next batch was added. After all the six batches were added, the reaction mixture was heated at reflux overnight, cooled down to room temperature, and then detected by LCMS to obtain the following results.

| Conversio n rate | Selectivity | Total yield | Yield of compound **16** (R=acetyl) | Yield of compound **16** (R=formyl) | Yield of compound **16** (R=H) |
|---|---|---|---|---|---|
| **96.7%** | **45.3%** | **43.8%** | **30.5%** | **12.3%** | **1.0%** |

### Example 12

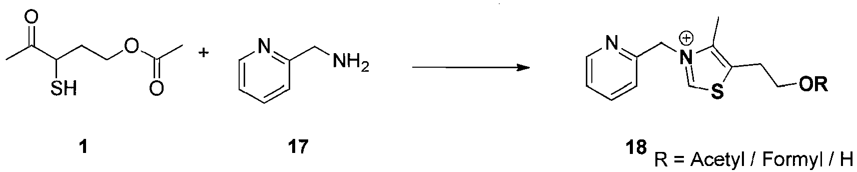

Under the protection of nitrogen, **34** mmol of compound **17** and **100** ml of formic acid were added into a three-necked flask equipped with a stirring device and heated to reflux. **42.7** mmol of compound **1** was added in six batches, and each batch was reacted for **1** hour before a next batch was added. After all the six batches were added, the reaction mixture was heated at reflux overnight, cooled down to room temperature, and then detected by LCMS to obtain the following results.

| Conversio n rate | Selectivity | Total yield | Yield of compound **18** (R=acetyl) | Yield of compound **18** (R=formyl) | Yield of compound **18** (R=H) |
|---|---|---|---|---|---|
| **39.6%** | **73.2%** | **28.9%** | **14.3%** | **3.4%** | **11.2%** |

### Example 13

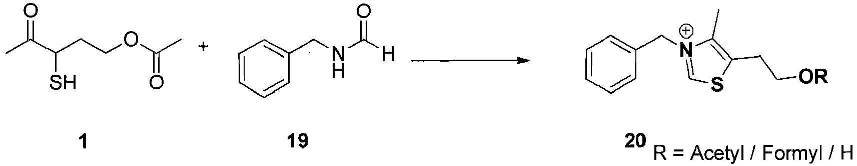

Under the protection of nitrogen, **14.5** mmol of compound **19** and **50** ml of formic acid were added into a three-necked flask equipped with a stirring device and heated to reflux. **24.1** mmol of compound **1** was added in six batches, and each batch was reacted for **1** hour before a next batch was added. After all the six batches were added, the reaction mixture was refluxed overnight, cooled down to room temperature, and then detected by LCMS to obtain the following results.

| Conversio n rate | Selectivity | Total yield | Yield of compound **20** (R=acetyl) | Yield of compound **20** (R=formyl) | Yield of compound **20** (R=H) |
|---|---|---|---|---|---|
| **83.2%** | **9.6%** | **8.0%** | **6.1%** | **1.1%** | **0.8%** |

### Example 14

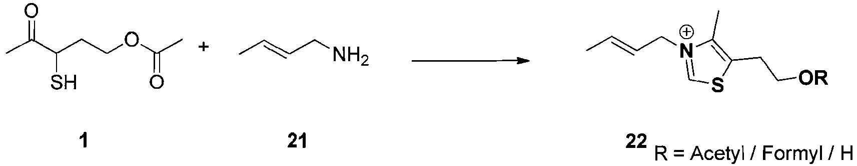

Under the protection of nitrogen, **45** mmol of compound **21** and **100** ml of formic acid were added into a three-necked flask equipped with a stirring device and heated to reflux. **67** mmol of compound **1** was added in six batches, and each batch was reacted for **1** hour before a next batch was added. After all the six batches were added, the reaction mixture was refluxed overnight, cooled down to room temperature, and then detected by LCMS to obtain the following results.

| Conversio n rate | Selectivity | Total yield | Yield of compound **22** (R=acetyl) | Yield of compound **22** (R=formyl) | Yield of compound **22** (R=H) |
|---|---|---|---|---|---|
| **54.0%** | **18.5%** | **10.0%** | **8.9%** | **1.1%** | **0%** |

### Example 15

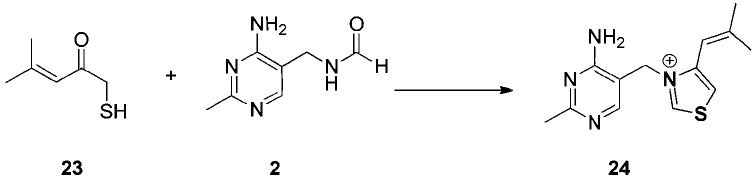

Under the protection of nitrogen, **8.3** mmol of compound **2** and **10** ml of formic acid were added into a three-necked flask equipped with a stirring device and heated to reflux. **8.3** mmol of compound **23** was added in three batches, and each batch was reacted for **1.5** hours before a next batch was added. After all the three batches were added, the reaction mixture was refluxed overnight, cooled down to room temperature, and then detected by LCMS liquid chromatography to obtain the following results.

| Conversion rate | Selectivity | Total yield | Yield of compound **24** |
|---|---|---|---|
| **19.0%** | **59.3%** | **11.3%** | **11.3%** |

### Example 16

After completion of the reaction, the reaction mixture obtained in Example **1** was evaporated to remove formic acid. The residue was added **300** ml of water to filter off insoluble impurities. The filtrate was added **262.5** mmol hydrochloric acid solution (**37%**) and then **7.5** g activated carbon with stirring, and then heated at **60**°C for **2** hours. After the active carbon was filtrated out, the filtrate was concentrated to about **50%** of the concentration. The obtained solution was added dropwise into **400** ml ethanol at **60**°C**,** stirred for **0.5** hours, cooled down to room temperature, and then stilled in refrigerator (**2-4**°C) for **3** hours. After filtration and washing with cold ethanol, the obtained solid was dried at **40**°C under vacuum to give **40.4** g of thiamine hydrochloride.

## Claims

1. A method for the preparation of a thiazole compound of formula (I), 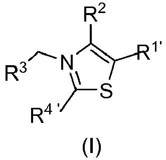 comprising reacting a compound of formula (II) with a compound of formula (III) in a solvent containing formic acid, 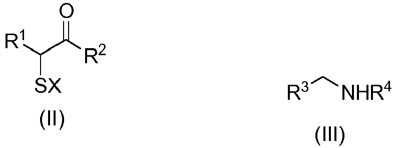 wherein,
each of R¹, R^{1'} and R² is independently hydrogen, or substituted or unsubstituted C₁-C₁₀ hydrocarbyl;
R³ is H, or substituted or unsubstituted C₁-C₁₀ hydrocarbyl, 5- or 6-members heterocyclic group or 5- or 6-members heteroaryl;
R⁴ is hydrogen, or substituted or unsubstituted C₁-C₁₀ acyl;
R⁴ is hydrogen, or substituted or unsubstituted C₁-C₁₀ hydrocarbyl; and
X is H, C₁-C₁₀ acyl, or the following structure: 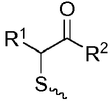 and,
wherein the process does not use any phosphorus-containing catalyst or any water absorbent.

2. The method of claim 1, wherein each of R¹ and R^{1'} is independently C₁-C₁₀ hydrocarbyl substituted by hydroxyl which is optionally substituted by C₁-C₁₀ acyl.

3. The method of claim 1, wherein R² is C₁-₁₀ alkyl.

4. The method of claim 1, wherein R³ is pyrimidinyl optionally substituted by C₁-C₁₀ hydrocarbyl such as methyl and/or amino group.

5. The method of claim 1, wherein R³ is 2-methyl-4-amino-5-pyrimidinyl.

6. The method of claim 1, wherein:
each of R¹ and R^{1'} is independently methyl, ethyl, propyl, ethenyl, hydroxyethyl, formyloxyethyl or acetyloxyethyl;
R² is methyl, ethyl, ethenyl, hydroxymethyl or hydroxyethyl;
R³ is phenyl, benzyl, 2-methyl-pyrimidinyl, 4-amino-pyrimidinyl, or 2-methyl-4-amino-pyrimidinyl;
R⁴ is H, formyl or acetyl;
R^{4'} is H or methyl; and
X is H, formyl, acetyl, or the following structure:

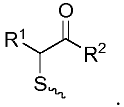

7. The method of any one of claims 1-6, wherein the solvent containing formic acid is formic acid or solvents mixture containing formic acid.

8. The method of claim 7, wherein the solvents mixture further contains acetic acid, dioxane and/or dimethylformamide.

9. The method of any one of claims 1-6, wherein the reaction is carried out at 80-150°C, preferably 100-120°C, more preferably at reflux temperature, under an atmosphere pressure or an increased pressure.

10. The method of any one of claims 1-6, wherein the molar ratio between the compound of formula (II) and the compound of formula (III) added into the reaction is from 1 : 2 to 2:1, preferably from 1:1 to 2:1, more preferably from 1:1 to 1.4: 1, and most preferably is 1.1:1.

11. The method of claim 1, wherein the compound of formula (II) and/or the compound of formula (III) are added into the reaction in batches or continuously.

12. The method of any one of claims 1-6, wherein the reaction is carried out in the presence or absence of a catalyst.

13. A method for the preparation of a thiazole compound of formula (Ia), 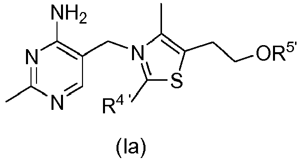 comprising reacting a compound of formula (IIa) with a compound of formula (IIIa) in a solvent containing formic acid, 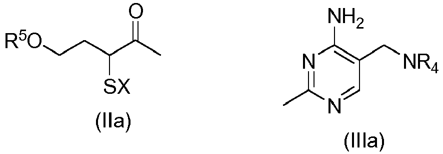
wherein R⁴ and R^{4'} are defined as any one of claims 1-8 and claim 20; and each of R⁵ and R^{5'} is independently hydrogen, formyl or acetyl, and,
wherein the process does not use any phosphorus-containing catalyst or any water absorbent.

14. A method for the preparation of a thiazole compound of formula (Ia), 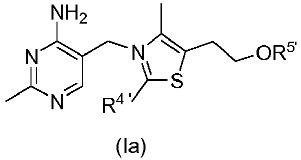 comprising reacting a compound of formula (IIb) with a compound of formula (IIIa) in a solvent containing formic acid, 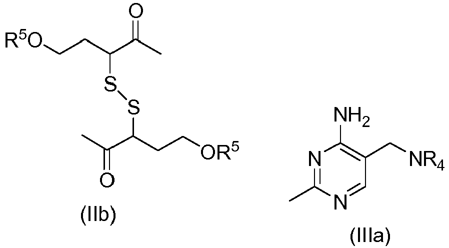
wherein, R⁴ and R^{4'} are defined as any one of claims 1-8 and claim 20; and each of R⁵ and R^{5'} is independently hydrogen, formyl or acetyl, and,
wherein the process does not use any phosphorus-containing catalyst.

## Patentansprüche

1. Verfahren zur Herstellung einer Thiazolverbindung der Formel (I), 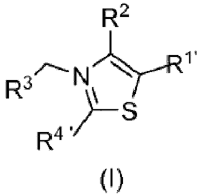 bei dem man eine Verbindung der Formel (II) mit einer Verbindung der Formel (III) in einem Ameisensäure enthaltenden Lösungsmittel umsetzt, 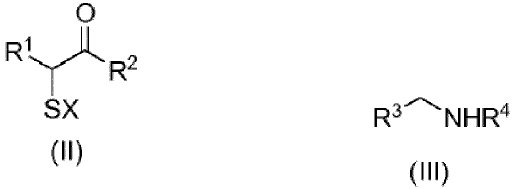 wobei
R¹, R^{1'} und R² jeweils unabhängig für Wasserstoff oder substituiertes oder unsubstituiertes C₁-C₁₀-Hydrocarbyl stehen;
R³ für H oder substituiertes oder unsubstituiertes C₁-C₁₀-Hydrocarbyl, eine 5- oder 6-gliedrige heterocyclische Gruppe oder 5- oder 6-gliedriges Heteroaryl steht;
R⁴ für Wasserstoff oder substituiertes oder unsubstituiertes C₁-C₁₀-Acyl steht;
R^{4'} für Wasserstoff oder substituiertes oder unsubstituiertes C₁-C₁₀-Hydrocarbyl steht; und
X für H, C₁-C₁₀-Acyl oder die folgende Struktur: 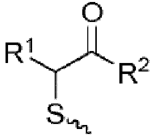 steht, und
wobei bei dem Verfahren kein phosphorhaltiger Katalysator oder Wasserabsorptionsmittel verwendet wird.

2. Verfahren nach Anspruch 1, bei dem R¹ und R^{1'} jeweils unabhängig für C₁-C₁₀-Hydrocarbyl, das durch Hydroxyl substituiert ist, das gegebenenfalls durch C₁-C₁₀-Acyl substituiert ist, stehen.

3. Verfahren nach Anspruch 1, bei dem R² für C₁-C₁₀-Alkyl steht.

4. Verfahren nach Anspruch 1, bei dem R³ für Pyrimidinyl, das gegebenenfalls durch C₁-C₁₀-Hydrocarbyl wie einer Methyl- und/oder Aminogruppe substituiert ist, steht.

5. Verfahren nach Anspruch 1, bei dem R³ für 2-Methyl-4-amino-5-pyrimidinyl steht.

6. Verfahren nach Anspruch 1, bei dem:
R¹ und R^{1'} jeweils unabhängig für Methyl, Ethyl, Propyl, Ethenyl, Hydroxyethyl, Formyloxyethyl oder Acyloxyethyl stehen;
R² für Methyl, Ethyl, Ethenyl, Hydroxymethyl oder Hydroxyethyl steht;
R³ für Phenyl, Benzyl, 2-Methylpyrimidinyl, 4-Aminopyrimidinyl oder 2-Methyl-4-aminopyrimidinyl steht;
R⁴ für H, Formyl oder Acetyl steht;
R^{4'} für H oder Methyl steht; und
X für H, Formyl, Acetyl oder die folgende Struktur: 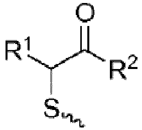 steht.

7. Verfahren nach einem der Ansprüche 1-6, bei dem es sich bei dem Ameisensäure enthaltenden Lösungsmittel um Ameisensäure oder eine Ameisensäure enthaltende Lösungsmittelmischung handelt.

8. Verfahren nach Anspruch 7, bei dem die Lösungsmittelmischung ferner Essigsäure, Dioxan und/oder Dimethylformamid enthält.

9. Verfahren nach einem der Ansprüche 1-6, bei dem die Umsetzung bei 80-150°C, vorzugsweise 100-120°C, weiter bevorzugt bei Rückflusstemperatur, unter Normaldruck oder erhöhtem Druck durchgeführt wird.

10. Verfahren nach einem der Ansprüche 1-6, bei dem das Molverhältnis zwischen der Verbindung der Formel (II) und der Verbindung der Formel (III), die zu der Umsetzung gegeben werden, 1:2 bis 2:1, vorzugsweise 1:1 bis 2:1, weiter bevorzugt 1:1 bis 1,4:1 und ganz besonders bevorzugt 1,1:1 beträgt.

11. Verfahren nach Anspruch 1, bei dem die Verbindung der Formel (II) und/oder die Verbindung der Formel (III) chargenweise oder kontinuierlich zu der Umsetzung gegeben werden.

12. Verfahren nach einem der Ansprüche 1-6, bei dem die Umsetzung in An- oder Abwesenheit eines Katalysators durchgeführt wird.

13. Verfahren zur Herstellung einer Thiazolverbindung der Formel (Ia), 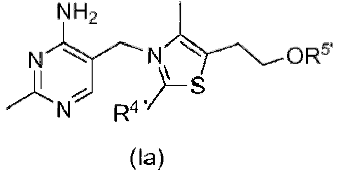 bei dem man eine Verbindung der Formel (IIa) mit einer Verbindung der Formel (IIIa) in einem Ameisensäure enthaltenden Lösungsmittel umsetzt, 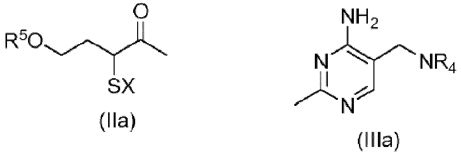
wobei R⁴ und R^{4'} wie in einem der Ansprüche 1-8 und Anspruch 20 definiert sind und R⁵ und R^{5'} jeweils unabhängig für Wasserstoff, Formyl oder Acetyl stehen, und
wobei bei dem Verfahren kein phosphorhaltiger Katalysator oder Wasserabsorptionsmittel verwendet wird.

14. Verfahren zur Herstellung einer Thiazolverbindung der Formel (Ia), 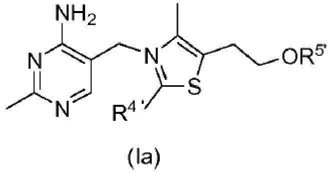 bei dem man eine Verbindung der Formel (IIb) mit einer Verbindung der Formel (IIIa) in einem Ameisensäure enthaltenden Lösungsmittel umsetzt, 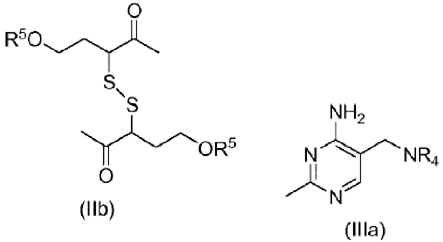
wobei R⁴ und R^{4'} wie in einem der Ansprüche 1-8 und Anspruch 20 definiert sind und R⁵ und R^{5'} jeweils unabhängig für Wasserstoff, Formyl oder Acetyl stehen, und
wobei bei dem Verfahren kein phosphorhaltiger Katalysator verwendet wird.

## Revendications

1. Procédé de synthèse d'un thiazole de formule (I), 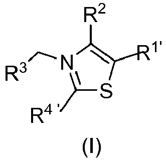 comprenant la réaction d'un composé de formule (II) avec un composé de formule (III) dans un solvant contenant de l'acide formique, 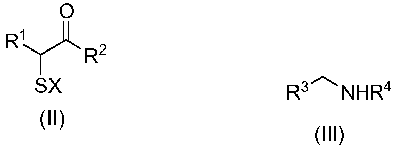 où,
chacun des radicaux R¹, R^{1'} et R² représente indépendamment un atome d'hydrogène ou un groupement hydrocarbyle en C₁-C₁₀ substitué ou non substitué ;
R³ représente H ou un groupement hydrocarbyle en C₁-C₁₀ substitué ou non substitué, hétérocyclique à 5 ou 6 chaînons ou hétéroaryle à 5 ou 6 chaînons ;
R⁴ représente un atome d'hydrogène ou un groupement acyle C₁-C₁₀ substitué ou non substitué ;
R^{4'} représente un atome d'hydrogène ou un groupement hydrocarbyle C₁-C₁₀ substitué ou non substitué ; et
X représente H ou un groupement acyle en C₁-C₁₀ répondant à la structure suivante : 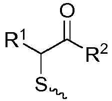 et, où le procédé n'utilise aucun catalyseur phosphoré ni absorbant d'eau.

2. Procédé selon la revendication 1, où chacun des radicaux R¹ et R^{1'} représente indépendamment un groupement hydrocarbyle en C₁-C₁₀ substitué par hydroxyle qui est éventuellement substitué par acyle en C₁-C₁₀.

3. Procédé selon la revendication 1, où R² représente un groupement alkyle en C₁-C₁₀

4. Procédé selon la revendication 1, où R³ représente un groupement pyrimidinyle éventuellement substitué par hydrocarbyle en C₁-C₁₀, tel qu'un groupement méthyle et/ou amino.

5. Procédé selon la revendication 1, où R³ représente un groupement 2-méthyl-4-amino-5-pyrimidinyle.

6. Procédé selon la revendication 1, ou
chacun des radicaux R¹ et R^{1'} représente indépendamment un groupement méthyle, éthyle, propyle, éthényle, hydroxyéthyle, formyloxyéthyle ou acétyloxyéthyle ;
R² représente un groupement méthyle, éthyle, éthényle, hydroxyméthyle ou hydroxyéthyle ;
R³ représente un groupement phényle, benzyle, 2-méthyl-pyrimidinyle, 4-amino-pyrimidinyle ou 2-méthyl-4-amino-pyrimidinyle ;
R⁴ représente H ou un groupement formyle ou acétyle ; R^{4'} représente H ou un groupement méthyle ; et
X représente H ou un groupement formyle, acétyle ou répondant à la structure suivante :

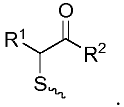

7. Procédé selon l'une quelconque des revendications 1 à 6, où le solvant contenant l'acide formique est l'acide formique ou un mélange de solvants contenant de l'acide formique.

8. Procédé selon la revendication 7, où le mélange de solvants comporte en outre de l'acide acétique, du dioxane et/ou du diméthylformamide.

9. Procédé selon l'une quelconque des revendications 1 à 6, où la réaction est mise en oeuvre entre 80 à 150 °C, préférentiellement entre 100 et 120 °C, plus préférentiellement à la température de reflux, à pression atmosphérique ou sous une pression accrue.

10. Procédé selon l'une quelconque des revendications 1 à 6, où le rapport molaire entre le composé de formule (II) et le composé de formule (III) ajouté dans la réaction est compris entre 1:2 et 2:1, préférentiellement entre 1:1 et 2:1, et préférentiellement entre 1:1 et 1,4:1, et le plus préférentiellement est égal à 1,1:1.

11. Procédé selon la revendication 1, où le composé de formule (II) et/ou le composé de formule (III) sont ajoutées dans la réaction par lots ou de façon continue.

12. Procédé selon l'une quelconque des revendications 1 à 6, où la réaction est mise en oeuvre en présence ou en l'absence d'un catalyseur.

13. Procédé de synthèse d'un thiazole de formule (Ia), 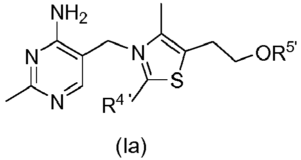 comprenant la réaction d'un composé de formule (IIa) avec un composé de formule (IIIa) dans un solvant contenant de l'acide formique, 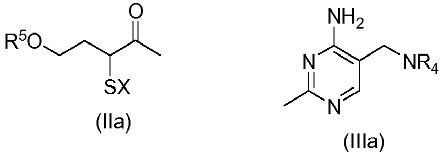
où R⁴ et R^{4'} sont tels que définis dans l'une quelconque des revendications 1 à 8 et la revendication 20 ; et chacun des radicaux R⁵ et R^{5'} représente indépendamment un atome d'hydrogène ou un groupement formyle ou acétyle, et
où le procédé n'utilise aucun catalyseur phosphoré ni absorbant d'eau.

14. Procédé de synthèse d'un thiazole de formule (Ia), 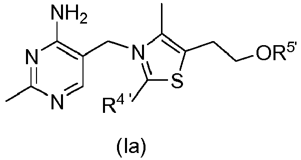 comprenant la réaction d'un composé de formule (IIb) avec un composé de formule (IIIa) dans un solvant contenant de l'acide formique, 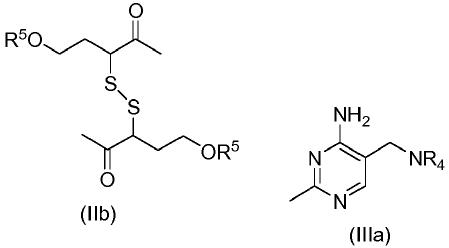
où R⁴ et R^{4'} sont tels que définis dans l'une quelconque des revendications 1 à 8 et la revendication 20 ; et chacun des radicaux R⁵ et R^{5'} représente indépendamment un atome d'hydrogène ou un groupement formyle ou acétyle, et
où le procédé n'utilise aucun catalyseur phosphoré.
